(19) Europäisches Patentamt
European Patent Office
Office européen des brevets

(11) EP 1 671 582 A1

(12) **EUROPEAN PATENT APPLICATION**

(43) Date of publication:
**21.06.2006 Bulletin 2006/25**

(51) Int Cl.:
***A61B 5/053** (2006.01)*

(21) Application number: **05025787.2**

(22) Date of filing: **25.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **16.12.2004 JP 2004364810**

(71) Applicant: **TANITA CORPORATION**
**Tokyo (JP)**

(72) Inventors:
- **Iijima, Aki**
  **Itabashi-ku, Tokyo (JP)**
- **Fukuda, Yoshinori**
  **Itabashi-ku, Tokyo (JP)**

(74) Representative: **Grünecker, Kinkeldey, Stockmair & Schwanhäusser Anwaltssozietät**
**Maximilianstrasse 58**
**80538 München (DE)**

(54) **Female physical condition managing apparatus**

(57) Disclosed is a female physical condition managing apparatus, which comprises input means for entering a bioelectric impedance-related parameter value, storage means for storing a time series of the parameter values entered for a given time period, change-trend acquisition means for acquiring a parameter-value change trend in accordance with the time-series parameter values, and physical condition estimation/determination means for estimating and determining a woman-specific cyclically-occurring change in physical condition, and a change in physical condition occurring independently of the cycle, in accordance with the change trend. The female physical condition managing apparatus of the present invention is capable of reliably estimating and determining a change in physical condition which is felt by a subject, irrespective of a menstrual cycle, and is useful in making an action plan in subject's daily life.

FIG. 7

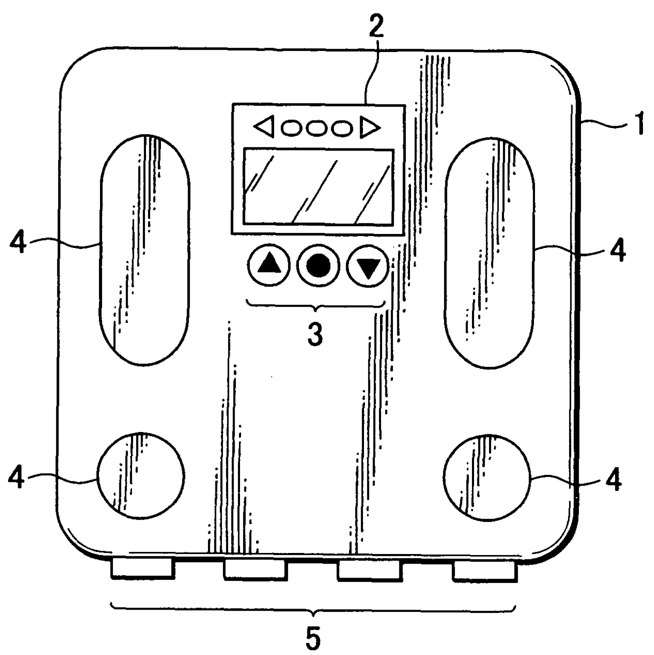


EP 1 671 582 A1

## Description

TECHNICAL FIELD

The present invention relates to a female physical condition managing apparatus for managing a female physical condition by means of bioelectric impedance.

BACKGROUND ART

As is known in regard to female physical conditions, a cyclic change in physical condition occurs in conjunction with a menstrual cycle. In particular, symptoms observed just before a menstrual period are called PMS (Premenstrual Syndrome), and women are more likely to feel a disorder of the body in this time period. From this point of view, the clarification of a female menstrual cycle is regarded as valuable in managing a female physical condition. As one physical condition management technique, a basal body temperature method is commonly known which comprises managing a physical condition in accordance with a menstrual cycle determined from a relationship between a female menstrual cycle and a change in basal body temperature.

As another technique for managing a change in physical condition occurring in conjunction with a menstrual cycle, a female physical condition managing apparatus has been proposed which is designed to evaluate and manage a physical condition along with a menstrual cycle, in accordance with a change in bioelectric impedance value (see, for example, the following Patent Publication 1).

[Patent Publication] Japanese Patent Laid-Open Publication No. 2001-78977

DISCLOSURE OF THE INVENTION

The above conventional management technique based on a menstrual cycle determined through the basal body temperature method is primarily intended for birth control, and originally involves difficulty in clarifying a menstrual cycle and expressing a change in physical conditions. For example, a basal body temperature can be accurately measured only if the measurement is performed under specific conditions, such as a specific measurement time in the morning or a specific measurement posture. This is liable to cause a mismatch between a change in basal body temperature and a menstrual cycle, or an increase of invisible change in body temperature. Thus, it is originally difficult to individually clarify menstrual cycles of all females, and thereby it is really hard to express a change in physical conditions.

While the conventional physical condition management techniques based on a determined menstrual cycle, including the female physical condition managing apparatus as disclosed in the Patent Publication 1, can simply determine and estimate a change in physical conditions depending on a menstrual cycle, they cannot additionally figure out and express a change in physical conditions occurring independently of the menstrual cycle.

Moreover, the sensory perception of a physical condition varies between individuals, and it is often the case that some women have indefinite complaints which are hard to be verbalized, and some women cannot find such symptoms until someone else mentions it. Most of the conventional techniques for evaluating a physical condition depending on a menstrual cycle are simply designed to indicate a physical condition which is considered as symptoms typically occurring during a specific time period in a menstrual cycle, but not designed to express a physical condition which is being felt by a subject.

It is therefore an object to provide a female physical condition managing apparatus capable of reliably estimating a woman-specific cyclic change in physical condition and a change in physical condition occurring independently of the cycle.

In order to achieve the above object, the present invention provides a female physical condition managing apparatus which comprises input means for entering a bioelectric impedance-related parameter value, storage means for storing a time series of the parameter values entered for a given time period, change-trend acquisition means for acquiring a parameter-value change trend in accordance with the time-series parameter values, and physical condition estimation/determination means for estimating and determining a woman-specific cyclically-occurring change in physical condition, and a change in physical condition occurring independently of the cycle, in accordance with the change trend.

According to the female physical condition managing apparatus, a change in physical condition which is felt by a subject can be estimated and determined irrespective of a menstrual cycle. Thus, the female physical condition managing apparatus of the present invention is useful in making an action plan in subject's daily life.

In the female physical condition managing apparatus of the present invention, the bioelectric impedance-related parameter value may be at least one selected from the group consisting of the absolute value of a bioelectric impedance, a reactance component value of the bioelectric impedance and a resistance component value of the bioelectric impedance.

In this case, conventional bioelectric impedance measurement techniques can be readily incorporated into the apparatus of the present invention, and advantages of such conventional techniques can be reflected directly in the apparatus of the present invention

In the female physical condition managing apparatus of the present invention, the change-trend acquisition means may include reference-condition-value calculation means for calculating a reference condition value in accordance with one or more of the time-series parameter values which are indicative of a past condition serving as a criterion for the change trend, current-condition-value calculation means for calculating a current condition value in accordance with one or more of the time-series parameter values which are indicative of a current condition, difference calculation means for calculating a difference between the reference condition value and the current condition value, and condition-change-amount calculation means for calculating a condition change amount per day in the calculated difference.

The above specific structure makes it possible to reduce an adverse affect of variation of measurement values so as to accurately express a change in physical condition from the past until now and clarify a relationship between a menstrual cycle and the change in physical condition.

The physical condition estimation/determination means may further include physical condition- estimation-index calculation means for calculating a physical condition estimation index to be used in estimating or determining a change in physical condition, in accordance with a change in sign of the condition-change amounts.

Further, the physical condition estimation/determination means may be operable, when the condition change amounts have a positive sign two times or more in succession, to estimate and determine that a current physical condition is good or is highly likely to be good within several days, and, when the condition change amounts have a negative sign two times or more in succession, to estimate and determine that a current physical condition is bad or is highly likely to be bad within several days.

According to the above specific structure, the change in physical condition can be presented to a subject in an easy-to-understand expression.

In the female physical condition managing apparatus of the present invention, the input means may include impedance measurement means for measuring a bioelectric impedance, and parameter-value acquisition means for acquiring a parameter value of the bioelectric impedance as the bioelectric impedance-related parameter value, and the female physical condition managing apparatus may further include measurement-date-data acquisition means for acquiring a measurement-date datum about date of the bioelectric impedance measurement. In this case, the storage means may be operable to store the time-series parameter values together with a time series of the measurement-date data associated, respectively, with the time-series of parameter values, and the change-trend acquisition means may be operable to acquire the parameter-value change trend in accordance with one of the time-series parameter values which is stored in association with the measurement-date datum closest to a predetermined time.

The above specific structure makes it possible to freely perform a daily measurement without being bound by a measurement time.

The female physical condition managing apparatus of the present invention may further include body-information input means for entering body information other than the bioelectric impedance-related parameter value, which is capable of exerting an influence on the bioelectric impedance-related parameter value, and correction means for correcting the bioelectric impedance-related parameter value in accordance with the body information.

According to the above specific structure, the change in physical condition can be estimated and determined in accordance with data having higher reliability.

Other features and advantages of the present invention will be apparent from the accompanying drawings and from the detailed description.

BRIEF DESCRIPTION OF DRAWINGS

FIG 1 is a chart showing a physical condition score indicative of the badness level of a female subjective physical condition.

FIG 2 is a graph showing a relationship between the absolute value of a bioelectric impedance and a physical condition score.

FIG 3 is a graph showing a relationship between a reactance component value of the bioelectric impedance and the physical condition score.

FIG 4 is a graph showing a relationship between a condition-value difference C and the physical condition score.

FIG 5 is a graph showing a relationship between a condition-change amount D and the physical condition score.

FIG 6 is a detailed explanatory graph of FIG 5.

FIG 7 is an external view showing a female physical condition managing apparatus according to one embodiment of the present invention

FIG 8 is a block diagram showing an electric system of the female physical condition managing apparatus according to the embodiment.

FIG 9 is a detailed diagram showing a display section of the female physical condition managing apparatus according to the embodiment.

FIG 10 is a flowchart showing a main routine to be performed in the female physical condition managing apparatus according to the embodiment.

FIG 11 is a flowchart showing a subroutine of a physical condition estimation/determination process.

FIG 12 is a flowchart showing a subroutine of a physical condition estimation-index calculation process.

FIG 13 is a chart showing a relationship between respective values of a physical condition estimation index.

FIG 14 is a detailed diagram showing one example of display in the display section.

FIG 15 is a block diagram showing a modification of the electric system, wherein the female physical condition managing apparatus according to the embodiment is designed to allow external connections.

BEST MODE FOR CARRYING OUT THE INVENTION

A female physical condition managing apparatus of the present invention comprises input means for entering a bioelectric impedance-related parameter value, storage means for storing a time series of the parameter values entered for a given time period, change-trend acquisition means for acquiring a parameter-value change trend in accordance with the time-series parameter values, and physical condition estimation/determination means for estimating and determining a woman- specific cyclically-occurring change in physical condition, and a change in physical condition occurring independently of the cycle, in accordance with the change trend.

The bioelectric impedance-related parameter value may be at least one selected from the group consisting of the absolute value of a bioelectric impedance, a reactance component value of the bioelectric impedance and a resistance component value of the bioelectric impedance.

The change-trend acquisition means may include reference-condition-value calculation means for calculating a reference condition value in accordance with one or more of the time-series parameter values which are indicative of a past condition serving as a criterion for the change trend, current-condition-value calculation means for calculating a current condition value in accordance with one or more of the time-series parameter values which are indicative of a current condition, difference calculation means for calculating a difference between the reference condition value and the current condition value, and condition-change-amount calculation means for calculating a condition change amount per day in the calculated difference.

The physical condition estimation/determination means may further include physical condition-estimation-index calculation means for calculating a physical condition estimation index to be used in estimating or determining a change in physical condition, in accordance with a change in sign of the condition-change amounts.

Further, the physical condition estimation/determination means may be operable, when the condition change amounts have a positive sign two times or more in succession, to estimate and determine that a current physical condition is good or is highly likely to be good within several days, and, when the condition change amounts have a negative sign two times or more in succession, to estimate and determine that a current physical condition is bad or is highly likely to be bad within several days.

The input means may include impedance measurement means for measuring a bioelectric impedance, and parameter-value acquisition means for acquiring a parameter value of the bioelectric impedance as the bioelectric impedance-related parameter value, and the female physical condition managing apparatus may further include measurement-date-data acquisition means for acquiring a measurement-date datum about date of the bioelectric impedance measurement. In this case, the storage means may be operable to store the time-series parameter values together with a time series of the measurement-date data associated, respectively, with the time-series of parameter values, and the change-trend acquisition means may be operable to acquire the parameter-value change trend in accordance with one of the time-series parameter values which is stored in association with the measurement-date datum closest to a predetermined time.

The female physical condition managing apparatus of the present invention may further include body-information input means for entering body information other than the bioelectric impedance-related parameter value, which is capable of exerting an influence on the bioelectric impedance-related parameter value, and correction means for correcting the bioelectric impedance-related parameter value in accordance with the body information.

In advance of the description of an embodiment of the present invention, the principle of estimation and determination of a female physical condition found by the inventors as the basis of the female physical condition managing apparatus of the present invention will be described with reference to FIGS. 1 to 6.

FIG 1 shows a part of physical condition scores for evaluating a subjective physical condition of a subject by a point rating. Specifically, with respect to each of items inductive of bad condition, such as "feels heavy in the breast" and "poor appetite", the level of badness is rated by a point, for example "slightly feel" = 1 point, "feel" = 2 and "strongly feel" = 3. The level of badness felt by the subject becomes higher as the total score of the points is increased.

Although not shown in the figures, a bioelectric impedance measurement device used in this measurement according to the female physical condition managing apparatus was a conventional four-electrode type designed to measure a bioelectric impedance using a plurality of frequencies under the condition that two current-supply electrodes and two voltage measurement electrodes are in contact with the subject's feet bottoms, and calculate a bioelectric impedance-related parameter value, such as the absolute value of the bioelectric impedance, a reactance component value of the bioelectric impedance and/or a resistance component value of the bioelectric impedance. In this measurement, a measurement time was set at an approximately the same time from day to day.

The following description will be made about the principle of estimation and determination of a physical condition in accordance with the result of an actual test for expressing the level of a subject's subjective physical condition indicated using the aforementioned physical condition scores, by a bioelectric impedance-related parameter.

FIG 2 is a typical example of a graph showing a relationship between the absolute values of bioelectric impedances measured at approximately the same time every day for about three months and the physical condition scores. The horizontal axis represents the number of days from a measurement initiation date. The absolute values of the bioelectric impedances are indicated by a heavy-line polygonal curve, wherein their measurement values are plotted by black circles, and the left vertical axis is used as a range axis thereof. The physical condition scores are indicated by a thin-line polygonal curve, wherein the scores are plotted by black squares, and the right vertical axis is used as a range axis thereof. In FIG 2, each hatched period corresponding to the days axis indicates a menstrual period.

FIG 3 is a graph showing a relationship between reactance component values calculated based on the bioelectric impedances and the physical condition scores. As with the absolute values of the bioelectric impedances in FIG 2, the reactance component values are indicated by a heavy-line polygonal curve, wherein the reactance component values are plotted by a black circle on a daily basis, and the left vertical axis is used as a range axis thereof.

In both of the graphs in FIGS. 2 and 3, the inventors found that, when the physical condition score is greater than zero, or when the subject feels a bad condition, a decreasing trend is observed in each of the absolute value and reactance component value of the bioelectric impedance on the current day or several days earlier. For example, in FIG 2, a relationship between a decrease of the absolute value of the bioelectric impedance and the physical condition score ranked as a bad condition can be remarkably observed in the relationship between the absolute value on around 10th day, which is surrounded by the dotted-line, and the physical condition score after several days therefrom, which is indicated by the black arrow.

In FIG 2, the physical condition score indicates a bad condition just before the hatched menstrual periods (around 20th day, around 50th day and around 80th day). This would show a female-specific cyclic change in physical condition, such as PMS, and it is proven that the absolute value of the bioelectric impedance also has a decreasing trend several days before each of the menstrual periods. In addition, as seen in the above relationship on around 10th day, it is proven that a change in physical condition occurring independently of the female-specific cycle is also expressed by the absolute value of the bioelectric impedance.

As seen in FIG 3, the above relationship in FIG 2 similarly appears in a relationship between the reactance component value and the physical condition score. Although not shown in the figures, a similar relationship could be observed in the relationship between the resistance component value and the physical condition score. Based on these facts, the following description will be made about the relationship between the reactance component value and the physical condition score illustrated in FIG 3, and additionally used as a substitute for the description about the relationship between the resistance component value and the physical condition score.

In the relationship illustrated in FIG 3, the absolute value of the bioelectric impedance and the physical condition score would include variation existing in each of the measurement values. Thus, it is difficult to estimate and determine a change of physical condition indicated by the he physical condition score, directly from the aforementioned reactance component value or the graph in FIG 3.

For this reason, the inventors developed a technique of acquiring a change trend in accordance with an average value of a time series of the reactance component values obtained for a given time period, and estimating and determining a change in physical condition in accordance with the acquired change trend. With reference to FIGS. 4 and 6, the estimation and determination of a physical condition in accordance with the reactance component value will be described in detail below by taking a specific example.

FIG 4 is a graph showing a relationship between condition-value differences C and the physical condition scores. The condition-value differences C are obtained as follows. Based on data about the reactance component values of the bioelectric impedances, a daily current condition value A was derived from previous three days' data including a datum associated with the latest measurement date, and a reference condition value B was derived from previous seven days' data including the datum associated with the latest measurement date. Then, each of the condition-value differences C was calculated by the following formula: the condition-value difference C = the current condition value A - the reference condition value B. In FIG 4, the condition-value differences C are indicated by a heavy-line polygonal curve, wherein their values are plotted by black circles, and the left vertical axis is used as a range axis thereof.

More specifically, the current condition value A is obtained by the following formula:

$$A = \{ \sum (Sn \times Wn)\} / ( \sum Wn)$$

wherein Sn is the reactance component value measured n-days before the latest measurement date, and Wn is a weighting factor for each of the reactance component values associated with the corresponding measurement dates. In this example, n was set to be 0, 1 and 2 to obtain the current condition value A from three days' data. Further, the weighting factor was set to be Wo = 3, $W_1$ = 2 and $W_3$ = 1, so as to allow the reactance component value associated with the latest measurement date to be reflected in the current condition value A.

Further, the reference condition value B is obtained by the following formula:

$$B = \{ \sum (Sm \times Wm)\} / ( \sum Wm)$$

wherein Sm is the reactance component value measured m-days before the latest measurement date, and Wm is a weighting factor for each of the reactance component values associated with the corresponding measurement dates. In this example, m was set to be 0 to 6 to obtain the current condition value A from seven days' data. Further, the weighting factor was set to be $W_0 = W_1 = 1$, $W_2 = W_3 = W_4 = 2$, and $W_5 = W_6 = 3$, so as to allow the reactance component values associated with the past measurement dates to be reflected in the reference condition value B.

For example, in FIG 4, a relationship between a decrease of the condition-value difference and the physical condition score ranked as a bad condition can be remarkably observed in the relationship between the condition-value difference on around 10th day, which is surrounded by the dotted-line, and the physical condition score after several days therefrom, which is indicated by the black arrow.

While n and m in this example were set, respectively, to 0 to 2 and 0 to 6 to obtain the current-condition value from previous three days' data and the reference condition value from previous seven day's data, and the weighting factors were set as above, these values are not limited to those in this example, except that m in the formula for the reference condition value B is essentially satisfy the following relation: m > n. Further, while m in this example was set to be 0 to 6 to use previous seven day's data for the reference condition value B, the number of days or a time period of the past data should be set to allow a change in physical condition occurring along with a menstrual cycle to be figured out. In this case, the time period is required to be set in a well balanced manner, because an excessively long period causes difficulty in detecting a change in physical condition occurring independently of the menstrual cycle, and an excessively short period is likely to cause the risk that variations of measurement values are included in the reference condition value B.

FIG 5 is a graph showing the relationship between a change in the condition-value differences C and the physical condition scores in an easy-to-understand manner, wherein the change in the condition-value differences C is expressed using condition change amounts D. In FIG 5, the condition change amounts D are indicated by a heavy-line polygonal curve, wherein the values are plotted by black circles, and the left vertical axis is used as a range axis thereof. Each of the condition change amounts D is obtained by the following formula:

(condition change amount D) = (condition-value difference on the current day) − (condition-value difference on the previous day)

That is, D < 0 means that the curve of the condition-value differences C illustrated in FIG 4 is changed to an upward or increasing trend. Conversely, D > 0 means that the curve of the condition-value differences C is changed to a downward or decreasing trend. Thus, a physical condition can be estimated and determined based on a relationship between signs (positive/negative signs) of the condition change amounts D and the physical condition scores. The relation between the change of the condition-value differences C and the physical condition scores for 30 days in FIG 5 is shown in FIG 6, wherein respective scales of the right and left vertical axes are changed to make it more understandable.

As seen in the region in FIG 6 surrounded by the dotted line, when the condition change amounts D have a negative sign for two days or more in succession, the physical condition score after several days therefrom, which is indicated by the tip of the black arrow, indicates a bad condition. Conversely, as seen in the region surrounded by the one-dot chain line, when the condition change amounts D have a positive sign for two days or more in succession, the physical condition score after several days therefrom, which is indicated by the tip of the black arrow, indicates a good condition (physical condition score = 0). As seen in FIG 5, this relationship can also be observed in the period after 30th day.

In the above way, based on the bioelectric impedance-related parameter value (reactance component value in the above example), a female-specific cyclic change in physical condition and a change in physical condition occurring independently of the female-specific cycle can be estimated and determined.

A female physical condition managing apparatus according to one embodiment of the present invention based on the aforementioned principle of estimating and determining a female physical condition in accordance with a reactance component value of a bioelectric impedance will be described below.

The female physical condition managing apparatus according to this embodiment is fundamentally designed using a conventional body-composition measurement apparatus adapted to measure bioelectric impedance and body weight so as to determine a body composition, such as body fat, muscle or body water. The following description will be made with reference to the drawings, on the assumption that the female physical condition managing apparatus according to this embodiment is used for a measurement once per day at a predetermined time of each day.

With reference to FIGS. 7 to 9, the structure of the female physical condition managing apparatus according to this embodiment will be firstly described below. FIG 7 is an external view showing a loading surface of the female physical condition managing apparatus, and FIG. 8 is a block diagram showing the configuration of an electric circuit of the female physical condition managing apparatus. FIG 8 is a detailed diagram showing a display section in FIG 7.

The female physical condition managing apparatus 1 illustrated in FIG 7 comprises a display section 2 for displaying an physical condition estimation/determination result, input/measurement/calculation results, and other information, an input section 3 for allowing a user or subject to enter therethrough his/her personal information necessary for a conventional body-composition measurement, such as age, sexuality and/or body height, a group of bioelectric-impedance measuring electrodes 4 to be in contact with feet bottoms during a bioelectric-impedance measurement, and a group of personal-registration/power switches 5 for allowing the subject to register or write the personal information and turn on power.

As seen in the block diagram of FIG 8, the display section 2 and the input section 3 are connected to a microcontroller 11. The microcontroller 11 is also connected with a body-weight measurement section 12, and a bioelectric-impedance measurement section 13 including the group of electrodes 4. The bioelectric-impedance measurement section 13 is operable to measure a bioelectric impedance of the subject using a plurality of frequencies. Further, a real-time clock 15 for measuring date is connected to the microcontroller 11.

The microcontroller 11 is a one-chip microcontroller internally including a CPU 16 and a storage section 16. The CPU 16 serves as a control section for controlling each section of the female physical condition managing apparatus, a conventional calculation section for calculating a reactance component value of the bioelectric impedance, and a physical condition estimation/determination section for estimating and determining a female physical condition in accordance with the reactance component value. The storage section 17 is composed of a RAM and a ROM, and adapted to store the personal information, measurement and calculation results, physical condition estimation/determination results and other information.

Referring to FIG 9, the display section 2 includes a physical condition display region 6 for indicating a female physical condition estimation/determination result, and a measurement- information display region 7 for displaying the personal information entered from the input section, a body-weight value, a body-composition estimated value, date and other information. The physical condition display region 6 is composed of a group of LEDs 6a to 6e for indicating bad and good condition in a multistage manner. The LEDs 6a, 6b for indicating a bad condition are designed to emit red light, and the LED 6s is designed to emit yellow light. The LEDs 6d, 6e for indicating a good condition are designed to emit green light. When a physical condition is estimated or determined, a corresponding one of the LEDs will be turned on, as described later in connection with one display example. The measurement-information display region 7 is designed to display information in the same manner as that in a conventional body-composition measurement apparatus.

With reference to FIGS. 10 to 14, an operation of the female physical condition managing apparatus 1 will be described. FIG 10 is a flowchart showing a main routine to be performed in the female physical condition managing apparatus 1. FIG 11 is a flowchart showing a subroutine of a physical condition estimation/determination process, and FIG 12 is a flowchart showing a subroutine of a physical condition estimation-index calculation process. FIG 13 is a chart showing a relationship between respective values of a physical condition estimation index. FIG 14 shows one example of display in the display section 2.

In FIG 10, when either one of the personal-registration/power switches 5 is pushed to turn on power, it is determined whether the personal information is stored on a location of the storage section 17 corresponding to the pushed switch, i.e. whether a personal registration has already been done, in Step S1. When the determination in Step S1 is NO or no personal registration has been done, the process advances to Step S2 to perform a personal registration. Specifically, an initial value of each of age, sexuality and body height, which is displayed on the measurement-information display region 7 of the display section 2, is adjusted using up/down-keys, and the adjusted value is entered using a setup key. When, all of the items are entered, this personal information is stored on the corresponding location of the storage section, and the personal registration is completed. Then, the process advances to Step S3.

When the determination in Step S1 is YES or a personal registration has already been done, the process advances to Step S3. In Step S3, the registered personal information is read from the storage section 17, and the female physical condition managing apparatus 1 is initialized to carry out preparations for a measurement in Steps S4 and S5.

In Step S4, a body-weight measurement is performed by the body-weight measurement section in a conventional manner, and an obtained body-weight measurement datum is stored on a given location of the storage section 17. In Step S5, a bioelectric-impedance measurement is performed by the bioelectric-impedance measurement section in a conventional manner, and an obtained bioelectric-impedance measurement datum is stored on a given location of the storage section 17, as with the body-weight measurement datum.

Then, in Step S6, a body-composition, such as body fat, muscle and/or body water, is calculated by the conventional calculation section in the CPU 16 in accordance with values of the measured body weight, he measured bioelectric impedance and the entered personal information, and the calculated body-composition value is stored on the storage section 17 in the same manner as described above.

After completion of the bioelectric-impedance measurement and the body-composition calculation, the process advances to Step S7 to perform a physical condition estimation/determination process. In this physical condition estimation/determination process, a reactance component value is calculated from the measured bioelectric impedance, and a new index or physical condition estimation index F indicative of an estimated trend about a physical condition is acquired based on the aforementioned principle of physical condition estimation/determination, as described in detail later in connection with the flowchart in FIG 11.

After completion of the physical condition estimation index calculation, in Step S8, the above results are displayed on the physical condition display region 6 and the measurement-information display region 7 of the display section 2. Then, in Step S9, it is determined whether a predetermined display time has passed. If the determination in Step S9 is NO or the predetermined display time has not passed, the presses will return to Step S8 to continue the display. When the determination in Step S9 is YES or the predetermined display time has passed, the power supply is automatically turned off to terminate the process.

The physical condition estimation/determination process in Step S7 will be described with reference to the flowchart of FIG 11 showing a subroutine of the physical condition estimation/ determination process. When the process is shifted to this subroutine, a reactance component value of the bioelectric impedance, which is stored on the storage section 17 in Step S5 of FIG 10, is calculated by the conventional calculation section in the CPU 16, in Step S11. Concurrently, a date datum is acquired from the real-time clock 15, and the calculated reactance component value is stored on a given location of the storage section together with the date datum associated therewith. During this storing process, a counter for counting a measurement day in the CUP 16 sets a count value x to 0 (zero). That is, the counter is operable to associate the count value x = 0 to the latest reactance-component-value datum. Further, with respect to past reactance- component-value data stored on the storage section 17, the counter is operable, every time the latest datum is newly stored, to associate a count value x = 1 with datum stored one day ago, and associate a count value x = 2 with datum stored two days ago. In this manner, all count values of the past data will be updated.

The process of Step S12 and subsequent Steps is performed in the physical condition estimation/determination section in the CPC.

In Step S12, the past reactance-component-value data each having the updated count value are read from the storage section 17 together with the latest reactance-component-value datum. Then, in Step S 13, it is determined whether the number of the read data meets the requirement for calculating a reference condition value as described in the physical condition estimation/determination principle, or satisfies the relationship of $x \geq m$ (in this embodiment, m = 6, as with the aforementioned example). When the determination in Step S13 is YES or the relationship of $x \geq m$ is satisfied, the process advances to Step S14. In Step S14, a reference condition value B is calculated through the aforementioned process, and stored on a given location of the storage section 17.

If the determination in Step S 13 is NO or the relationship of $x \geq m$ is not satisfied and the number of the data is insufficient, the process will advance to Step S20. In Step S20, it is determined whether the number of the read data meets the requirement for calculating the current condition value A, or satisfies the relationship of $x > n$ (in this embodiment, n = 2, as with the aforementioned example). When the determination in Step S20 is YES or the relationship of $x > n$ is satisfied, the process advances to Step S21. In Step S21, the oldest one of the past data earlier than three days' data required for calculating the current condition value is temporarily designated as the reference condition value B, and stored on a given location of the storage section 17. If the determination in Step S20 is NO or $x \leq n$ and the number of the read data does not meet the requirement for calculating the reference condition value B, the process will advance to Step S22. In Step S22, an after-mentioned physical condition estimation index F is set to 0 (zero), and stored on a given location of the storage section. Then, the process returns to the main routine of FIG 10 without estimating/determining a physical condition.

As above, when the reference condition value B is stored on the storage section 17 in Steps S14 and S21, the current condition value A is calculated in Step S15 through the aforementioned process, and stored on a given location of the storage section 17.

Then, in Step S16, the aforementioned condition-value difference Cx is calculated in accordance with the stored current condition value A and reference condition value B, and stored on a given location of the storage section 17 in association with x = 0 or in the form of $C_0$.

Then, in Step S 17, it is determined whether a previous day's condition-value difference $C_1$ required for calculating the aforementioned condition change amount D is stored. If the determination in Step 17 is NO or no previous day's condition-value difference $C_1$ is stored, the process will advance to Step S22. In Step S22, the physical condition estimation index F is set to 0 (zero), and the process returns to the main routine, as with the Step S22. When the determination in Step 17 is YES or a previous day's condition-value difference $C_1$ is stored, the process advances to Step S 18. In Step S 18, a condition change amount D is calculated by the following formula: (condition change amount D) = (condition-value difference $C_0$) - (condition-value difference $C_1$), according to the aforementioned process, and stored on a given location of the storage section 17.

When the condition change amount D is obtained, the process is shifted from the Step S18 to a subroutine of an after-mentioned physical condition estimation-index calculation process. When the physical condition estimation index F indicative of an estimated trend about a physical condition (bad or good condition) is calculated based on the condition change amount D, the process returns from the subroutine to the main routine in FIG 10.

The physical condition estimation-index calculation process in the Step S 18 will be described in detail below with reference to the flowchart of FIG 12 showing the subroutine of the physical condition estimation-index calculation process. In the following description, a physical condition estimation index Fx associated with the latest measurement date or x = 0 is described as $F_0$, and a physical condition estimation index Fx associated with a previous day's measurement date or x = 1 is described as $F_1$.

Further, either one of four indexes of 0, 1, 2, 3 and 4 is assigned to the physical condition estimation-index F. Specifically, when the condition change amounts D have a positive sign for two days in succession, the index 0 is assigned to the F, and it is determined that "a physical condition is currently good or will be good within several days". Conversely, when the condition change amounts D have a negative sign for two days in succession, the index 3 is assigned to the F, and it is determined that "a physical condition is currently bad or will be bad within several days". When the index 1 or 2 is assigned to F, it is determined that a physical condition is likely, respectively, to become good or bad.

When the process is shifted to the subroutine, it is determined, in Step S31, whether the condition change amount D is equal to or greater 0 (zero). That is, it is determined whether a reactance component value associated with the latest measurement date has an increasing trend or a decreasing trend as compared with a previous day's reactance component value. When the determination in Step S31 is YES or $D \geq 0$ (zero) and it is determined that the physical condition has a tendency of improvement, the process advances to Step S32. In Step S32, a physical condition estimation index $F_0$ associated with the latest measurement date is obtained by the following formula: $F_0 = F_1 - 1$ (Formula I). In this the Formula I, even if the physical condition estimation index $F_1$ is not calculated in the subroutine of FIG 12, it is calculated in the form of $F_1 = 0$ (zero) which is set at the Step S22 of the physical condition estimation/determination process in FIG 11.

Then, in Step S33, it is determined whether the $F_0$ calculated by the Formula I is greater than 0 (zero). If the determination in Step S33 is NO or the $F_0$ is 0 (zero) or a negative value, the process will advance to Step S36. In Step S36, the index 0 is assigned to the $F_0$ or it is determined that "a physical condition is currently good or will be good within several days", and the process gets out of this subroutine.

When the determination in Step S33 is YES or the $F_0$ is a positive value, the process advances to Step S34. In Step S34, it is determined whether the $F_0$ is equal to 2. Specifically, when the $F_0$ has a positive value even after "1" is substituted from the physical condition estimation index $F_1$, $F_1$ must have the index 2 or 3 indicative of a possibility of a bad condition. However, the condition change amount is determined to be $D \geq 0$ in the Step S31, it may be considered that the physical condition has a trend of improvement. Thus, in order to avoid the situation where the calculation result in the Formula I is $F_0 = 3 - 1 = 2$, and thereby the physical condition is determined to be "likely to become bad", when the determination in Step S34 is YES or the calculation result in the Formula I is $F_0 = 2$, the process advances to Step S35 to assign the index 1 to the $F_0$ and determine that the physical condition is "likely to become good", and the process gets out of this subroutine. If the determination in Step S34 is NO or the calculation result in the Formula I is $F_0 \neq 2$, the process will directly get out of this subroutine, because the calculation result in the Formula I must be $F_0 = 1$.

When the determination in the Step S31 is NO or the state change amount $D < 0$ and it is determined that the physical condition has a tendency of decline, the process advances to Step S37. In Step S37, a physical condition estimation index $F_0$ associated with the latest measurement date is obtained by the following formula: $F_0 = F_1 + 1$ (Formula II).

Then, in Step S38, it is determined whether the $F_0$ calculated by the Formula II is equal to or greater than 3. When the determination in Step S38 is YES or the $F_0$ is equal to or greater than 3, the process advances to Step S41. In Step S41, the index 3 is assigned to the $F_0$, and it is determined that the physical condition is "currently bag or will be bad within several days". Then, the process will get out of this subroutine.

If the determination in Step S38 is NO or the $F_0 < 3$, the process will advance to Step S39. In Step S39, it is determined whether the $F_0$ calculated by the Formula II is equal to "1" indicative of a good condition, even through the determination in Step S31 is $D < 0$ indicative of a bad condition. When the determination in Step S39 is YES or $F_0 = 1$, the process advances to Step S40. In Step S40, the index 2 is assigned to the $F_0$ in place of "1", and it is determined that the physical condition is "likely to becomes bad". Then, the process will get out of this subroutine. If the determination in Step S39 is NO or $F_0 \neq 1$, the process will directly get out of this subroutine, because the calculation result in the Formula II must be $F_0 = 2$.

The above relationships are illustrated in the chart of FIG 13 in a view format.

FIG 14 shows one example of display in the physical condition display region 6 of the display section 2 when determination results based on the physical condition estimation index is displayed in the Step S8 of the flowchart showing the main routine illustrated in FIG 10. This display example shows a case where the index 3 is assigned to the physical condition estimation index F.

As described in connection with FIG 8, the physical condition display region 6 is composed of the group of LEDs 6a to 6e adapted to indicate bad and good conditions in a multistage manner. The LEDs 6a, 6b are designed to emit red light, and the LED 6s is designed to emit yellow light. The LEDs 6d, 6e are designed to emit green light. That is, each of the LEDs 6a to 6e is designed to be turned on in response to a corresponding one of the physical condition estimation indexes. For example, when the physical condition estimation index F = 3, the LEDs 6d, 6e indicative of a bad condition are turned on to call attention "a physical condition is currently bad or will be bad within several days" to a subj ect.

In the same way, when F = 2, the LEDs 6b (red), 6c (yellow) are turned on. When F = 1, the LEDs 6c (yellow), 6d (green) are turned on. Further, When F = 0, the LEDs 6d (green), 6e (green) are turned on.

The present invention has been described in connection with the above embodiment where the measurement using the female physical condition managing apparatus 1 is performed once per day at the same time. However, in real life, the measurement is likely to be performed several times in a day or to be skipped some times. In view of this situation, the apparatus may be designed such that, when the measurement is performed several times in a day, measurement data obtained at a time closest to a predetermined time may be selected using date data acquired from the real-time clock illustrated in FIG 9. Further, when there is a day having no measurement, irrespective of the number of days, at a time when data satisfying x = n and x = m are detected, the processes for calculating the current condition value A and the reference condition value B may be performed in the same manner as that in the above embodiment.

The estimation/determination of a physical condition in the above embodiment has been performed based on a reactance component value of a bioelectric impedance. In this case, variations in the body weight measured in the Step S4 of FIG 10 or the body composition calculated in the Step S6 of FIG 10 may be subjected to a correction processing to allow the physical condition to be more reliably estimated. For example, the bioelectric impedance and the body weight have a relationship that the bioelectric impedance has a smaller value as the body weight is increased, and the bioelectric impedance has a larger value is lowered as the body weight is reduced. Thus, in the Step S11 of FIG 11 showing the physical condition estimation/determination process in the Step S7 of FIG 10, before calculating a reactance component value of the bioelectric impedance, the bioelectric impedance may be corrected based on the relationship between the bioelectric impedance and the body weight.

Further, as shown in FIG. 15, the female physical condition managing apparatus 1 may be provided with an input/output interface circuit 20 capable of interfacing the apparatus with an external memory, a personal computer or the internet, to perform the physical condition management through an external terminal. For example, the external memory may include the aforementioned storage section 17 to store and read measured/calculated data through the input/output interface circuit 20. Further, the external memory may be designed to be detachable from the female physical condition managing apparatus 1 and portable so as to allow data measured in a remote location to be stored thereon. In this case, if the remote location is equipped with another female physical condition managing apparatus 1 implementation the present invention, the physical condition management utilizing the present invention can be performed without restriction on location. Furthermore, a management server may be provided to acquire various data from the personal computer or the like through the internet and manage the data. This makes it possible to collectively manage physical conditions of a plurality of subjects and provide appropriate advices to each subject. The management server may be provided with various calculation sections and physical condition estimation/ determination section in the aforementioned CPC 16, to establish a system for receiving measurement data and input data from each subject and performing various calculations and physical condition determinations in the management server.

## Claims

**1.** A female physical condition managing apparatus comprising:

input means for entering a bioelectric impedance-related parameter value;
storage means for storing a time series of said parameter values entered for a given time period;
change-trend acquisition means for acquiring a parameter-value change trend in accordance with said time-series parameter values; and
physical condition estimation/determination means for estimating and determining a woman-specific cyclically-occurring change in physical condition, and a change in physical condition occurring independently of said cycle, in accordance with said acquired change trend.

**2.** The female physical condition managing apparatus as defined in claim 1, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**3.** The female physical condition managing apparatus as defined in claim 1, wherein said change-trend acquisition means includes:

reference-condition-value calculation means for calculating a reference condition value in accordance with one or more of said time-series parameter values which are indicative of a past condition serving as a criterion for said change trend;
current-condition-value calculation means for calculating a current condition value in accordance with one or more of said time-series parameter values which are indicative of a current condition;
difference calculation means for calculating a difference between said reference condition value and said current condition value; and
condition-change-amount calculation means for calculating a condition change amount per day in said calculated difference.

**4.** The female physical condition managing apparatus as defined in claim 3, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**5.** The female physical condition managing apparatus as defined in claim 3, wherein said physical condition estimation/determination means includes physical condition-estimation-index calculation means for calculating a physical condition estimation index to be used in estimating or determining a change in physical condition, in accordance with a change in sign of said condition-change amounts.

**6.** The female physical condition managing apparatus as defined in claim 5, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time-series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**7.** The female physical condition managing apparatus as defined in claim 3, wherein said physical condition estimation/determination means is operable, when said condition change amounts have a positive sign two times or more in succession, to estimate and determine that a current physical condition is good or is highly likely to be good within several days, and, when said condition change amounts have a negative sign two times or more in succession, to estimate and determine that a current physical condition is bad or is highly likely to be bad within several days.

**8.** The female physical condition managing apparatus as defined in claim 7, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**9.** The female physical condition managing apparatus as defined in claim 1, wherein said bioelectric impedance-related parameter value is at least one selected from the group consisting of the absolute value of a bioelectric impedance, a reactance component value of the bioelectric impedance and a resistance component value of the bioelectric impedance.

**10.** The female physical condition managing apparatus as defined in claim 9, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**11.** The female physical condition managing apparatus as defined in claim 9, wherein said change-trend acquisition means includes:

reference-condition-value calculation means for calculating a reference condition value in accordance with one or more of said time-series parameter values which are indicative of a past condition serving as a criterion for the change trend;
current-condition-value calculation means for calculating a current condition value in accordance with one or more of said time-series parameter values which are indicative of a current condition;
difference calculation means for calculating a difference between said reference condition value and said current condition value; and
condition-change-amount calculation means for calculating a condition change amount per day in said calculated difference.

**12.** The female physical condition managing apparatus as defined in claim 11, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**13.** The female physical condition managing apparatus as defined in claim 11, wherein said physical condition estimation/ determination means includes physical condition-estimation-index calculation means for calculating a physical condition estimation index to be used in estimating or determining a change in physical condition, in accordance with a change in sign of said condition-change amounts.

**14.** The female physical condition managing apparatus as defined in claim 13, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**15.** The female physical condition managing apparatus as defined in claim 11, wherein said physical condition estimation/ determination means is operable, when said condition change amounts have a positive sign two times or more in succession, to estimate and determine that a current physical condition is good or is highly likely to be good within several days, and, when said condition change amounts have a negative sign two times or more in succession, to estimate and determine that a current physical condition is bad or is highly likely to be bad within several days.

**16.** The female physical condition managing apparatus as defined in claim 15, wherein said input means includes:

impedance measurement means for measuring a bioelectric impedance; and
parameter-value acquisition means for acquiring a parameter value of said bioelectric impedance as said bioelectric impedance-related parameter value,
wherein said female physical condition managing apparatus further includes measurement-date-data acquisition means for acquiring a measurement-date datum about date of said bioelectric impedance measurement, wherein:

said storage means is operable to store said time-series parameter values together with a time series of said measurement-date data associated, respectively, with said time-series of parameter values; and
said change-trend acquisition means is operable to acquire said parameter-value change trend in accordance with one of said time-series parameter values which is stored in association with the measurement-date datum closest to a predetermined time.

**17.** The female physical condition managing apparatus as defined in either one of claims 1 to 16, which further includes:

body-information input means for entering body information other than said bioelectric impedance-related parameter value, said body information being capable of exerting an influence on said bioelectric impedance-related parameter value; and
correction means for correcting said bioelectric impedance-related parameter value in accordance with said body information.

# FIG. 1

| CONDITION | PHYSICAL CONDITION | | | | | | | | | TOTAL |
|---|---|---|---|---|---|---|---|---|---|---|
| | HEAVY IN BREAST | PAIN IN BREAST | CONSTIPA-TION | HYPOSARCA | LISTLESS-NESS | POOR APPETITE | FATIGUE | SLEEPINESS | INACTIVITY | |
| SLIGHTLY FEEL | (1) | 1 | 1 | 1 | 1 | (1) | 1 | 1 | (1) | |
| FEEL | 2 | 2 | (2) | 2 | 2 | 2 | 2 | 2 | 2 | |
| STRONGLY FEEL | 3 | 3 | 3 | 3 | 3 | 3 | (3) | 3 | 3 | 8 |

FIG. 2

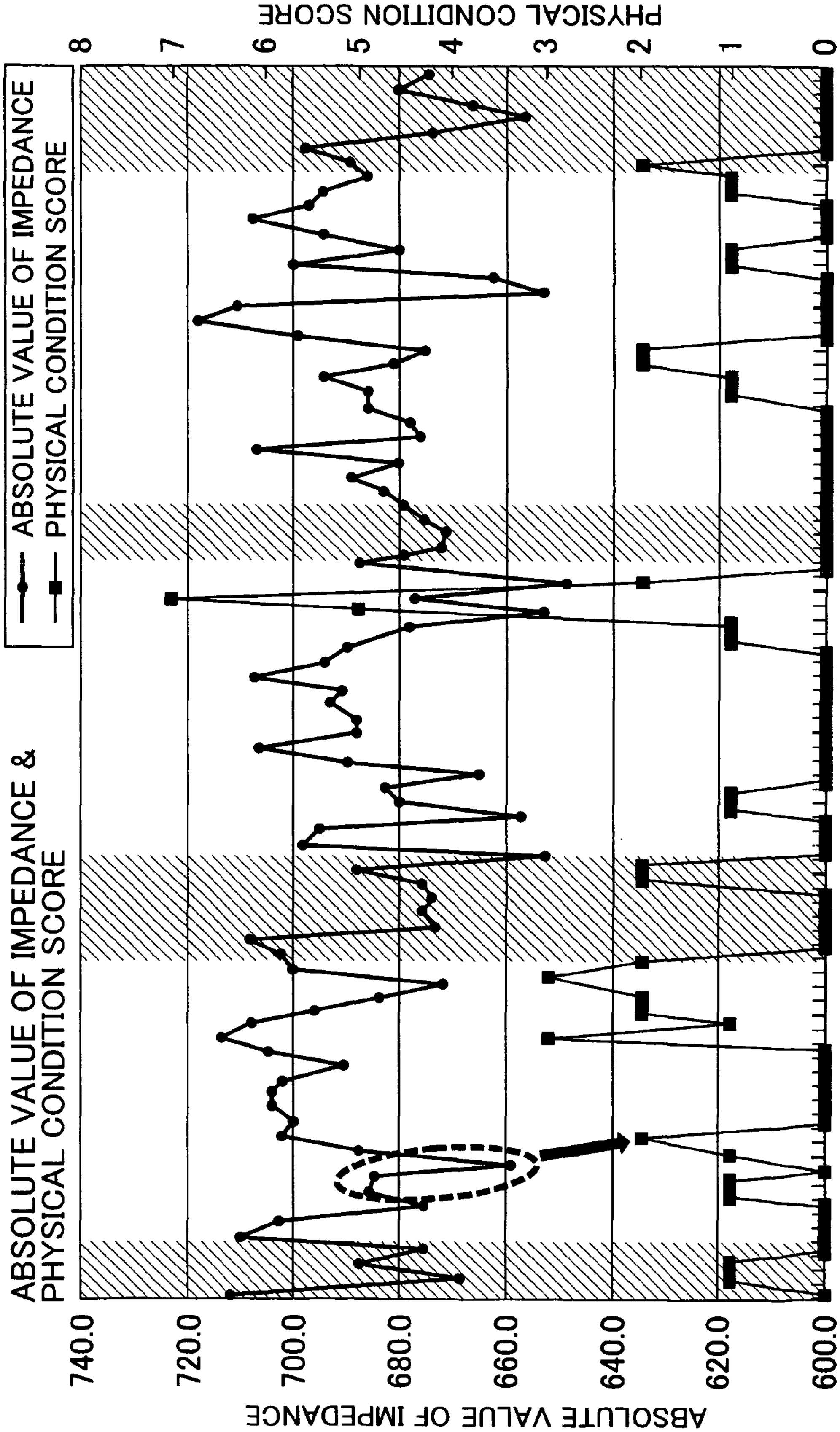
ABSOLUTE VALUE OF IMPEDANCE &
PHYSICAL CONDITION SCORE
ABSOLUTE VALUE OF IMPEDANCE
PHYSICAL CONDITION SCORE
ABSOLUTE VALUE OF IMPEDANCE
740.0
720.0
700.0
680.0
660.0
640.0
620.0
600.0
PHYSICAL CONDITION SCORE
8
7
6
5
4
3
2
1
0
DAYS
0
10
20
30
40
50
60
70
80

FIG. 3

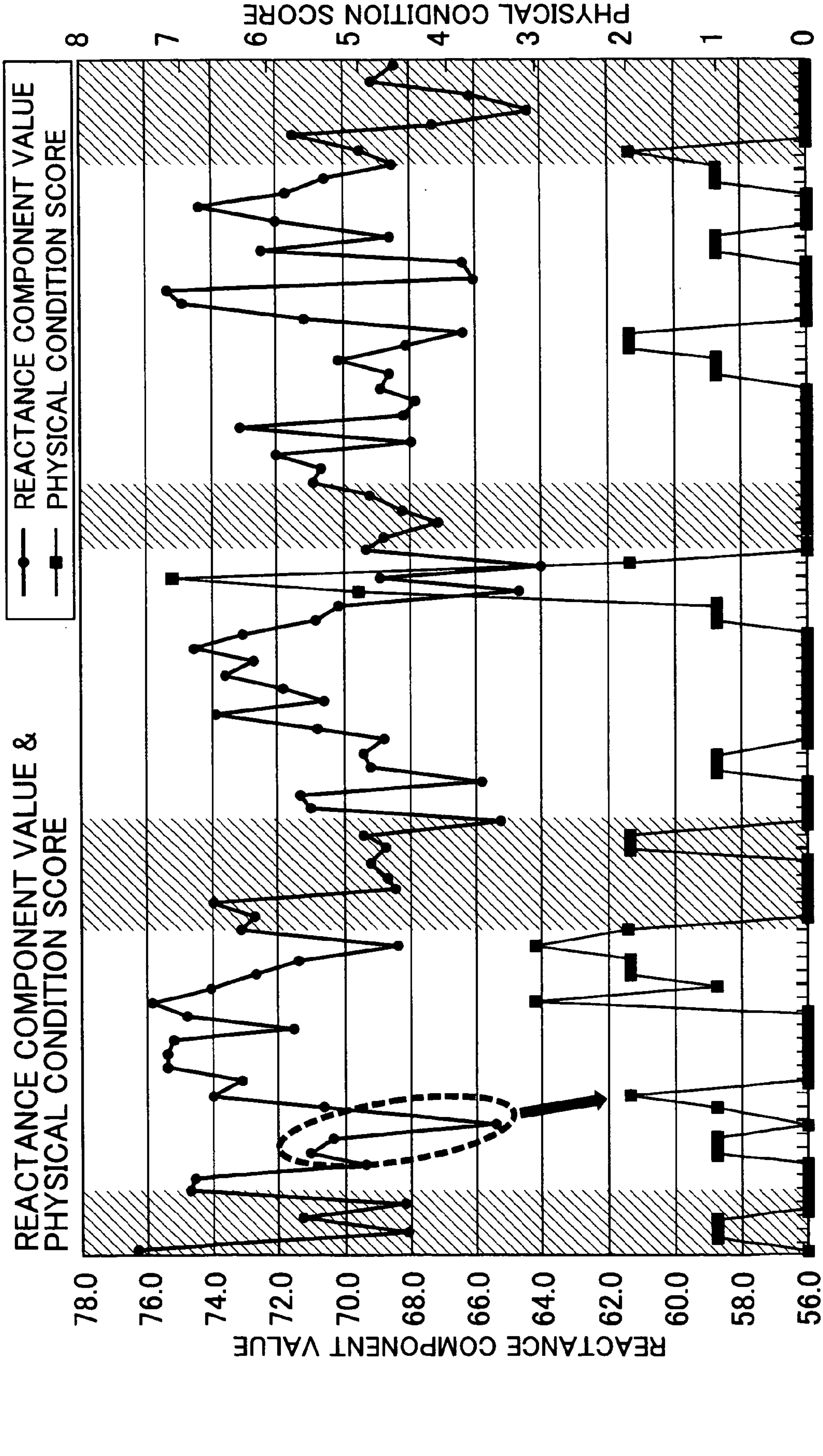
REACTANCE COMPONENT VALUE &
PHYSICAL CONDITION SCORE
REACTANCE COMPONENT VALUE
PHYSICAL CONDITION SCORE
REACTANCE COMPONENT VALUE
PHYSICAL CONDITION SCORE
DAYS
78.0
76.0
74.0
72.0
70.0
68.0
66.0
64.0
62.0
60.0
58.0
56.0
0
10
20
30
40
50
60
70
80
8
7
6
5
4
3
2
1
0

FIG. 4

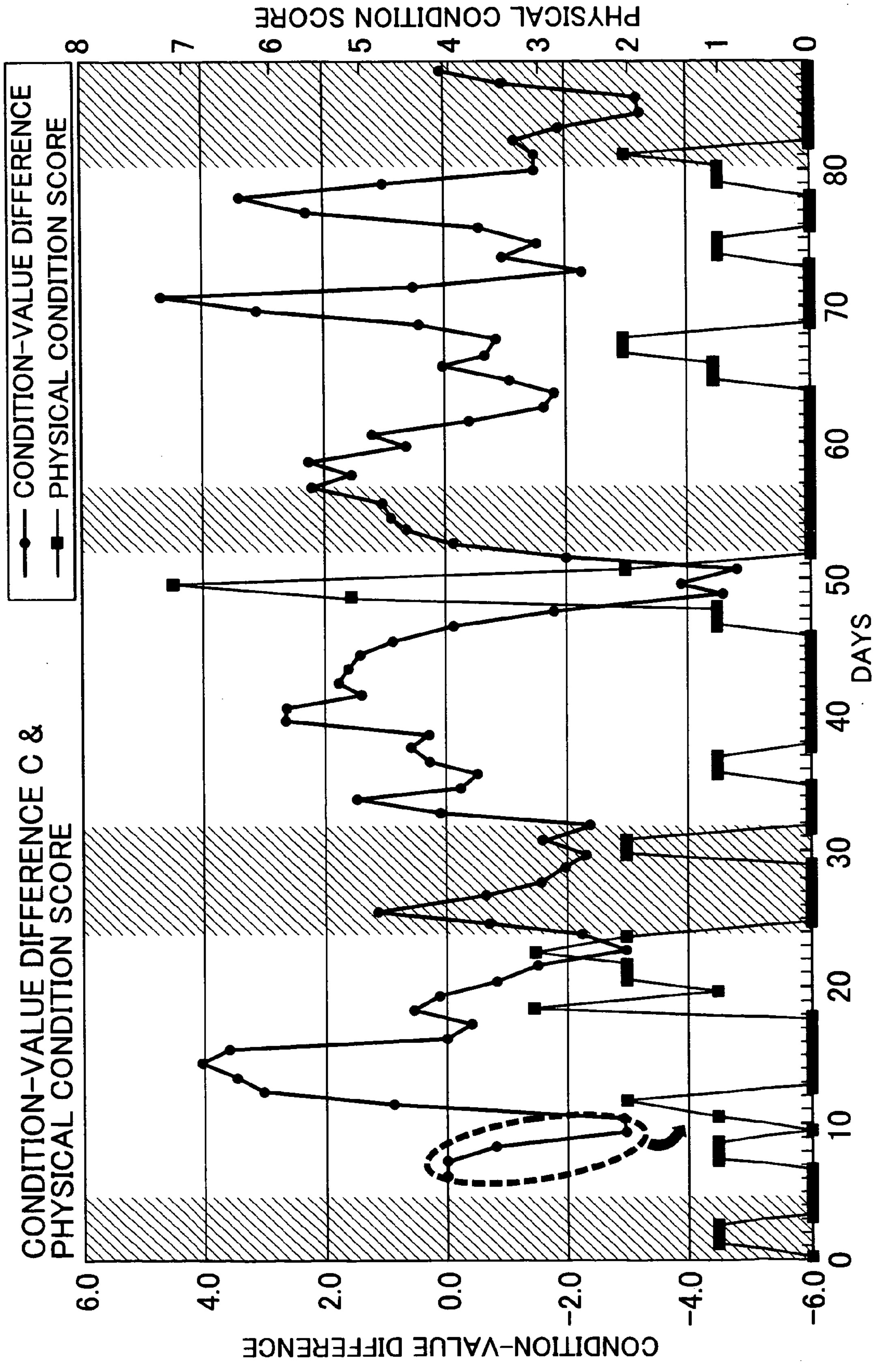
CONDITION-VALUE DIFFERENCE C &
PHYSICAL CONDITION SCORE
CONDITION-VALUE DIFFERENCE
PHYSICAL CONDITION SCORE
CONDITION-VALUE DIFFERENCE
PHYSICAL CONDITION SCORE
DAYS
6.0
4.0
2.0
0.0
-2.0
-4.0
-6.0
0
1
2
3
4
5
6
7
8
0
10
20
30
40
50
60
70
80

FIG. 5

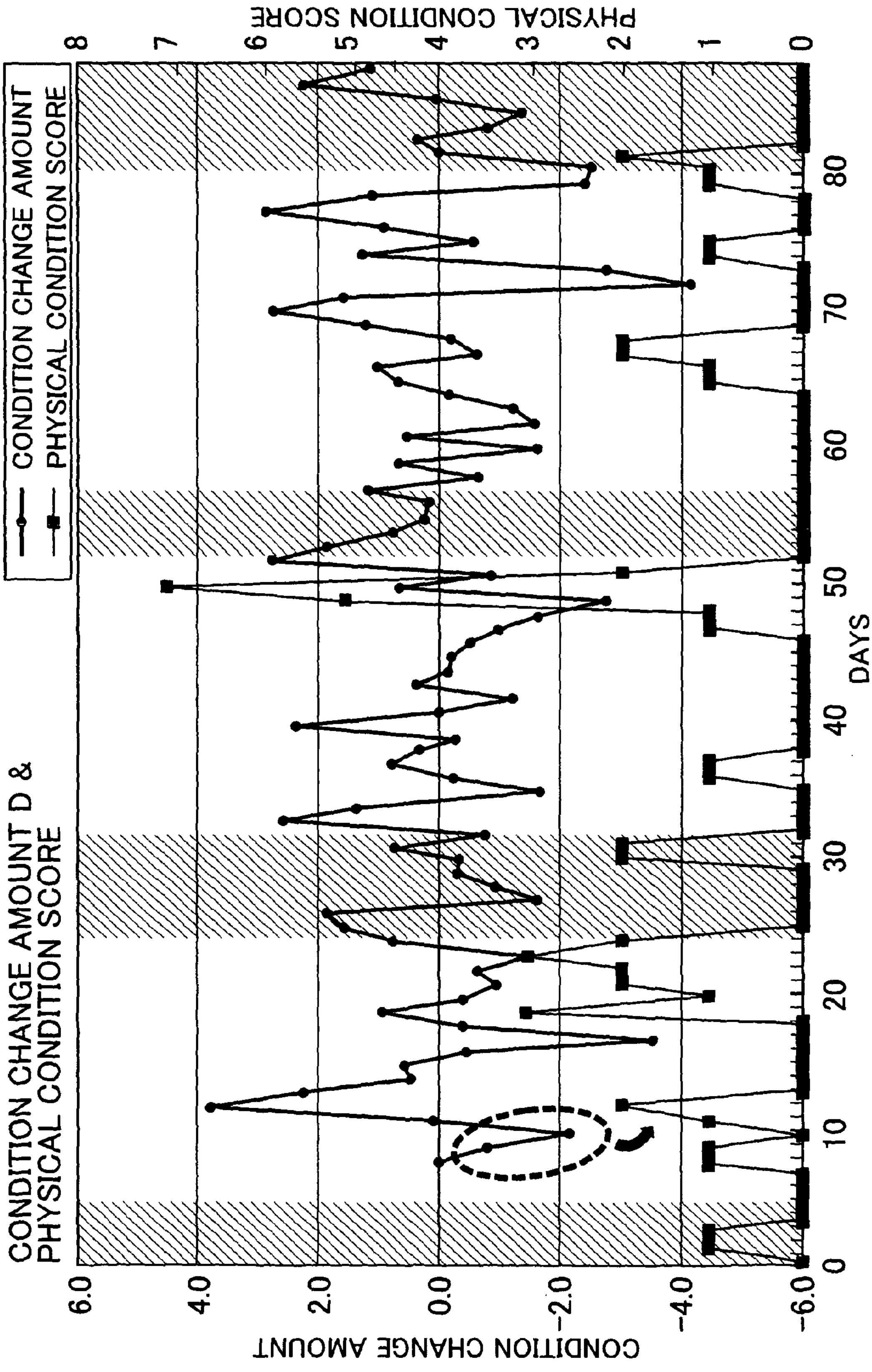
CONDITION CHANGE AMOUNT D &
PHYSICAL CONDITION SCORE
CONDITION CHANGE AMOUNT
PHYSICAL CONDITION SCORE
CONDITION CHANGE AMOUNT
PHYSICAL CONDITION SCORE
DAYS
6.0
4.0
2.0
0.0
-2.0
-4.0
-6.0
0
10
20
30
40
50
60
70
80
8
7
6
5
4
3
2
1
0

FIG. 6

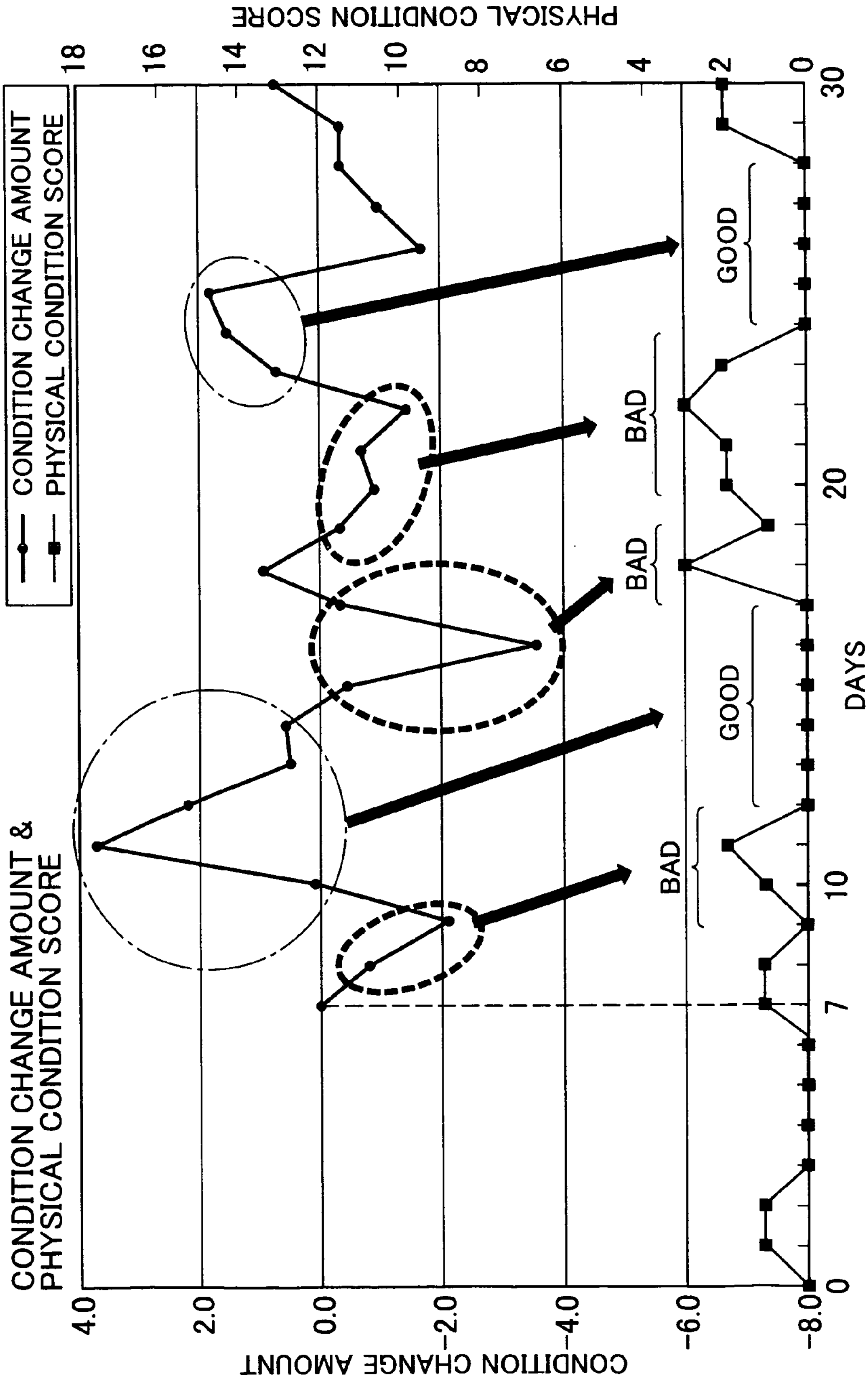
CONDITION CHANGE AMOUNT &
PHYSICAL CONDITION SCORE
CONDITION CHANGE AMOUNT
PHYSICAL CONDITION SCORE
CONDITION CHANGE AMOUNT
4.0
2.0
0.0
-2.0
-4.0
-6.0
-8.0
PHYSICAL CONDITION SCORE
18
16
14
12
10
8
6
4
2
0
DAYS
0
7
10
20
30
BAD
GOOD
BAD
BAD
GOOD

FIG. 7

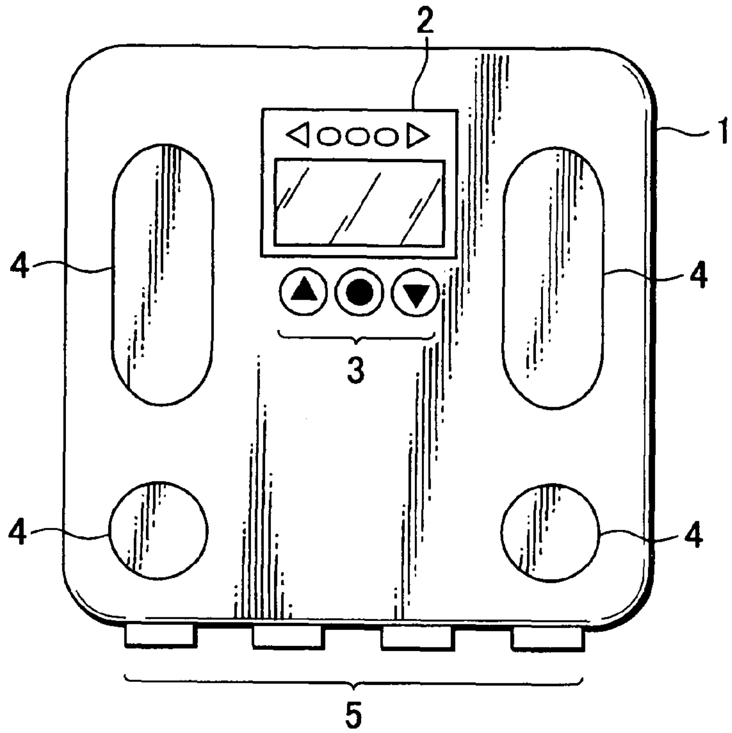
1
2
3
4
4
4
4
5

FIG. 8

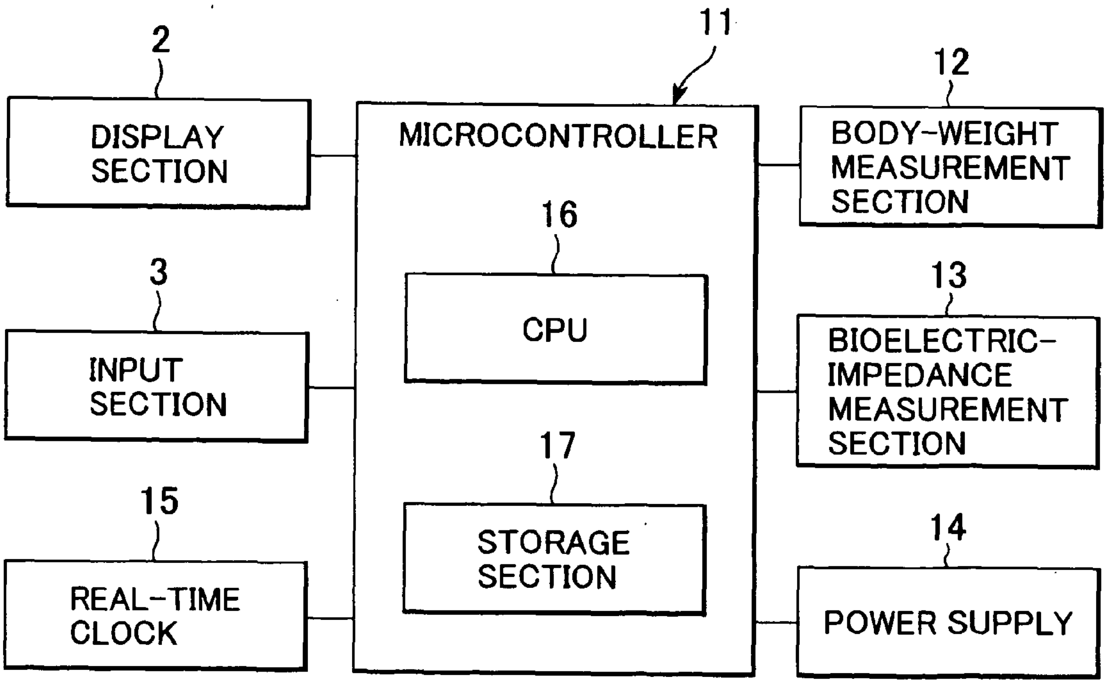
2
DISPLAY SECTION
3
INPUT SECTION
15
REAL-TIME CLOCK
11
MICROCONTROLLER
16
CPU
17
STORAGE SECTION
12
BODY-WEIGHT MEASUREMENT SECTION
13
BIOELECTRIC-IMPEDANCE MEASUREMENT SECTION
14
POWER SUPPLY

FIG. 9

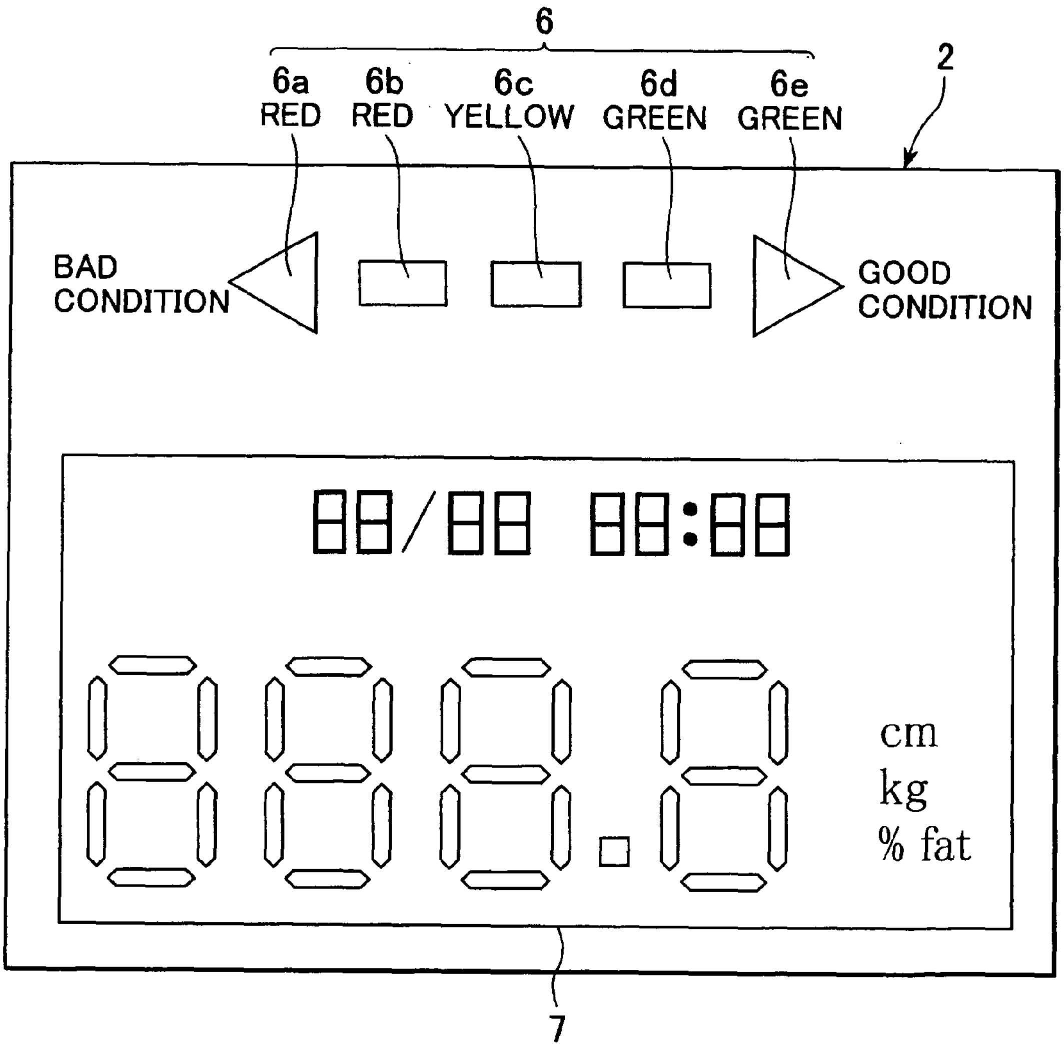
6
6a
RED
6b
RED
6c
YELLOW
6d
GREEN
6e
GREEN
2
BAD CONDITION
GOOD CONDITION
88/88 88:88
cm
kg
% fat
7

FIG. 10

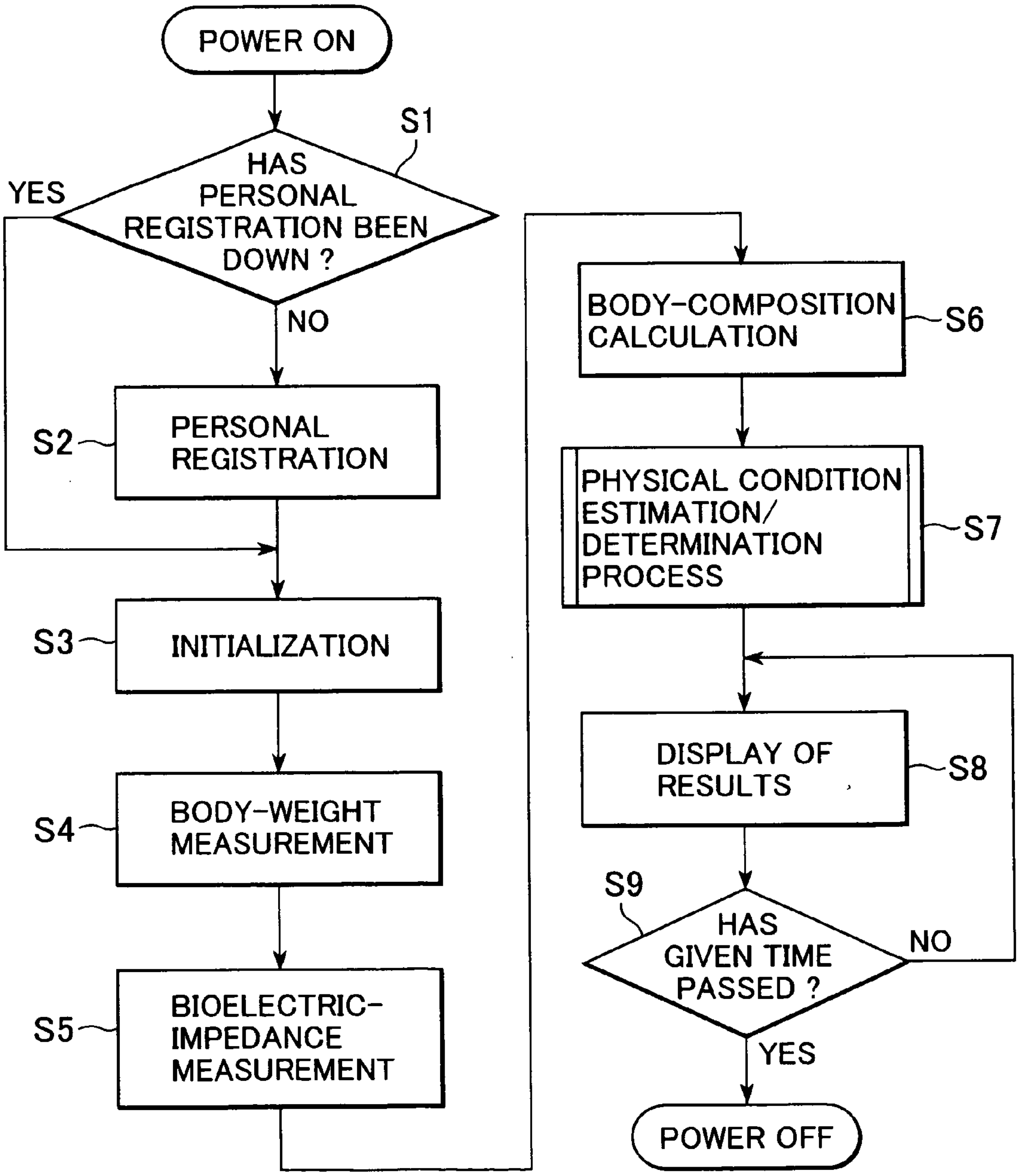
POWER ON
S1
HAS PERSONAL REGISTRATION BEEN DOWN ?
YES
NO
S2
PERSONAL REGISTRATION
S3
INITIALIZATION
S4
BODY-WEIGHT MEASUREMENT
S5
BIOELECTRIC-IMPEDANCE MEASUREMENT
BODY-COMPOSITION CALCULATION
S6
PHYSICAL CONDITION ESTIMATION/ DETERMINATION PROCESS
S7
DISPLAY OF RESULTS
S8
S9
HAS GIVEN TIME PASSED ?
NO
YES
POWER OFF

FIG. 11

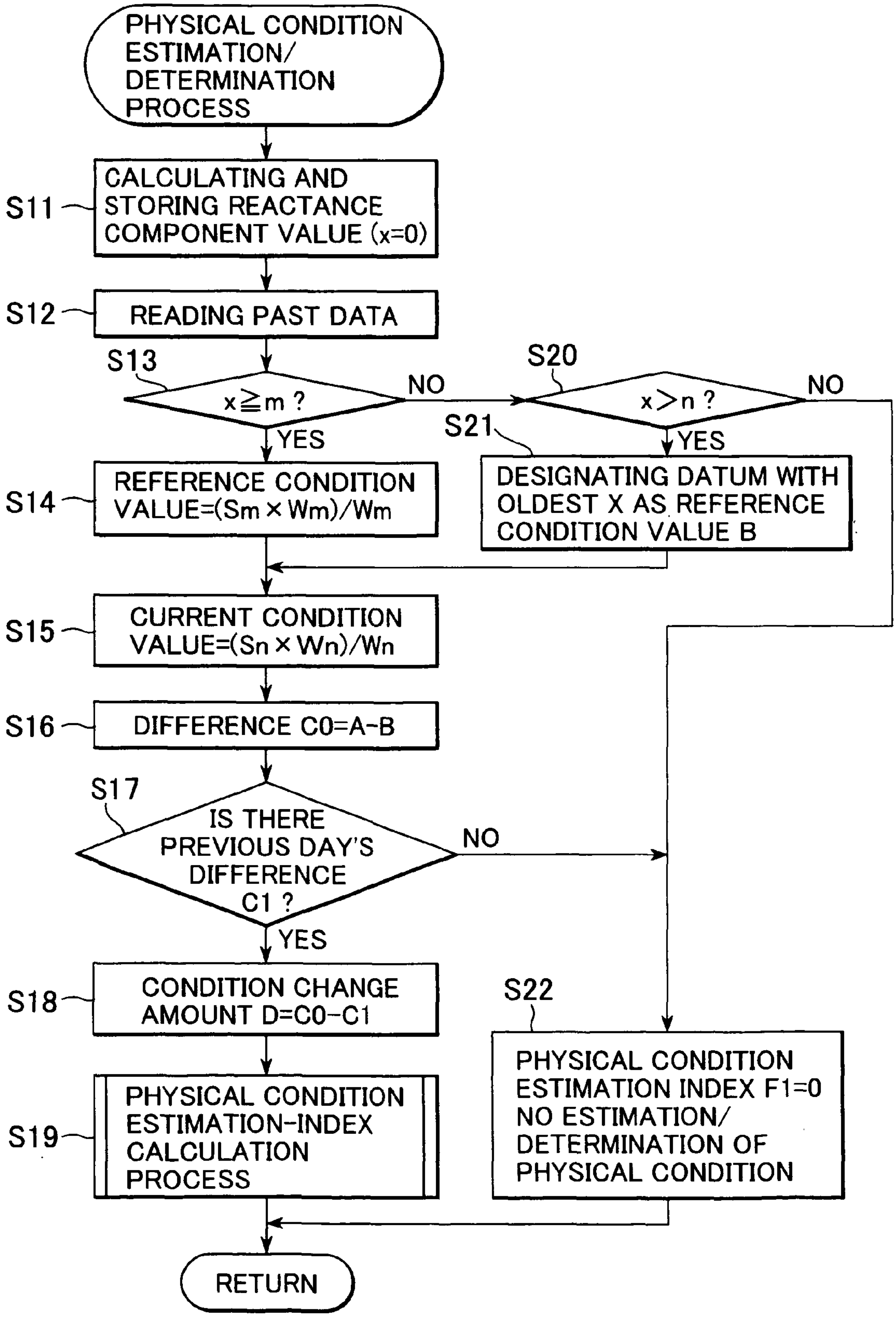
PHYSICAL CONDITION ESTIMATION/ DETERMINATION PROCESS
S11
CALCULATING AND STORING REACTANCE COMPONENT VALUE (x=0)
S12
READING PAST DATA
S13
x≧m ?
NO
YES
S14
REFERENCE CONDITION VALUE=(Sm × Wm)/Wm
S20
x>n ?
NO
YES
S21
DESIGNATING DATUM WITH OLDEST X AS REFERENCE CONDITION VALUE B
S15
CURRENT CONDITION VALUE=(Sn × Wn)/Wn
S16
DIFFERENCE C0=A-B
S17
IS THERE PREVIOUS DAY'S DIFFERENCE C1 ?
NO
YES
S18
CONDITION CHANGE AMOUNT D=C0-C1
S19
PHYSICAL CONDITION ESTIMATION-INDEX CALCULATION PROCESS
S22
PHYSICAL CONDITION ESTIMATION INDEX F1=0 NO ESTIMATION/ DETERMINATION OF PHYSICAL CONDITION
RETURN

FIG. 12

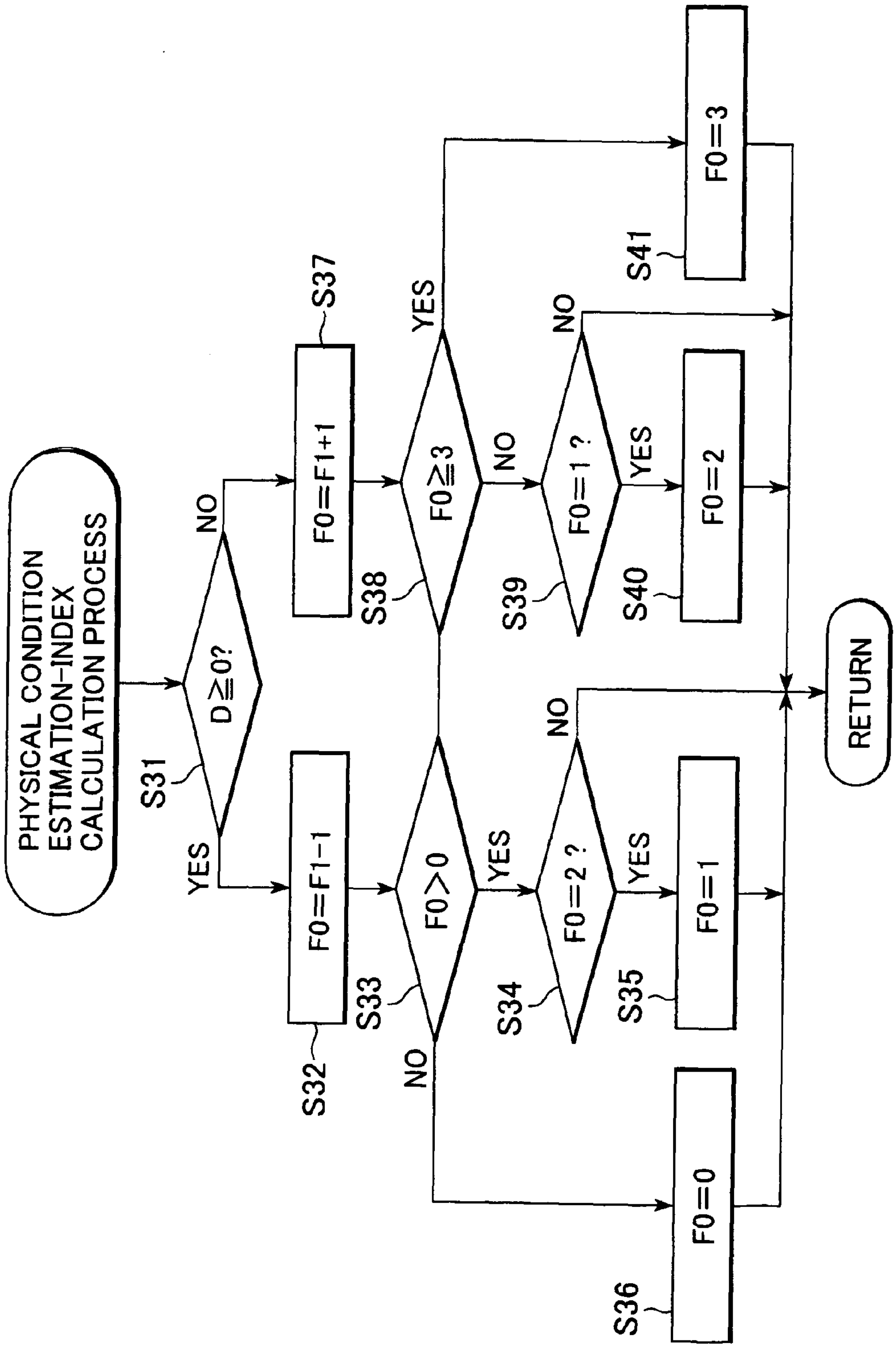
PHYSICAL CONDITION ESTIMATION-INDEX CALCULATION PROCESS
S31
D≧0?
YES
NO
S32
F0=F1−1
S37
F0=F1+1
S33
F0>0
S38
F0≧3
YES
NO
S34
F0=2 ?
S39
F0=1 ?
S35
F0=1
S40
F0=2
S36
F0=0
S41
F0=3
RETURN

FIG. 13

| F1 | D | F0 |
|---|---|---|
| 0 | D≧0 | 0 |
| | D<0 | 2 |
| 1 | D≧0 | 0 |
| | D<0 | 2 |
| 2 | D≧0 | 1 |
| | D<0 | 3 |
| 3 | D≧0 | 1 |
| | D<0 | 3 |

FIG. 14

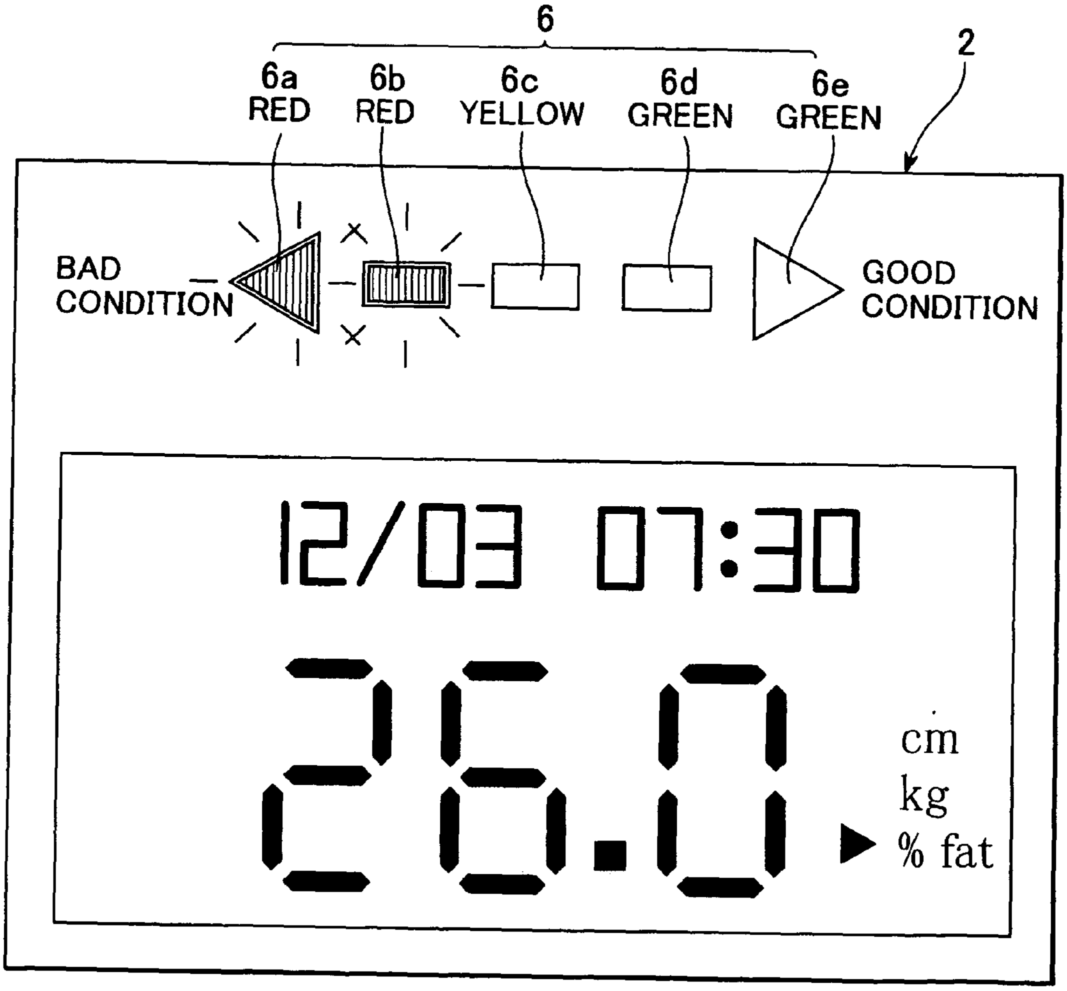
6
2
6a
RED
6b
RED
6c
YELLOW
6d
GREEN
6e
GREEN
BAD
CONDITION
GOOD
CONDITION
12/03 07:30
26.0
cm
kg
% fat

FIG. 15

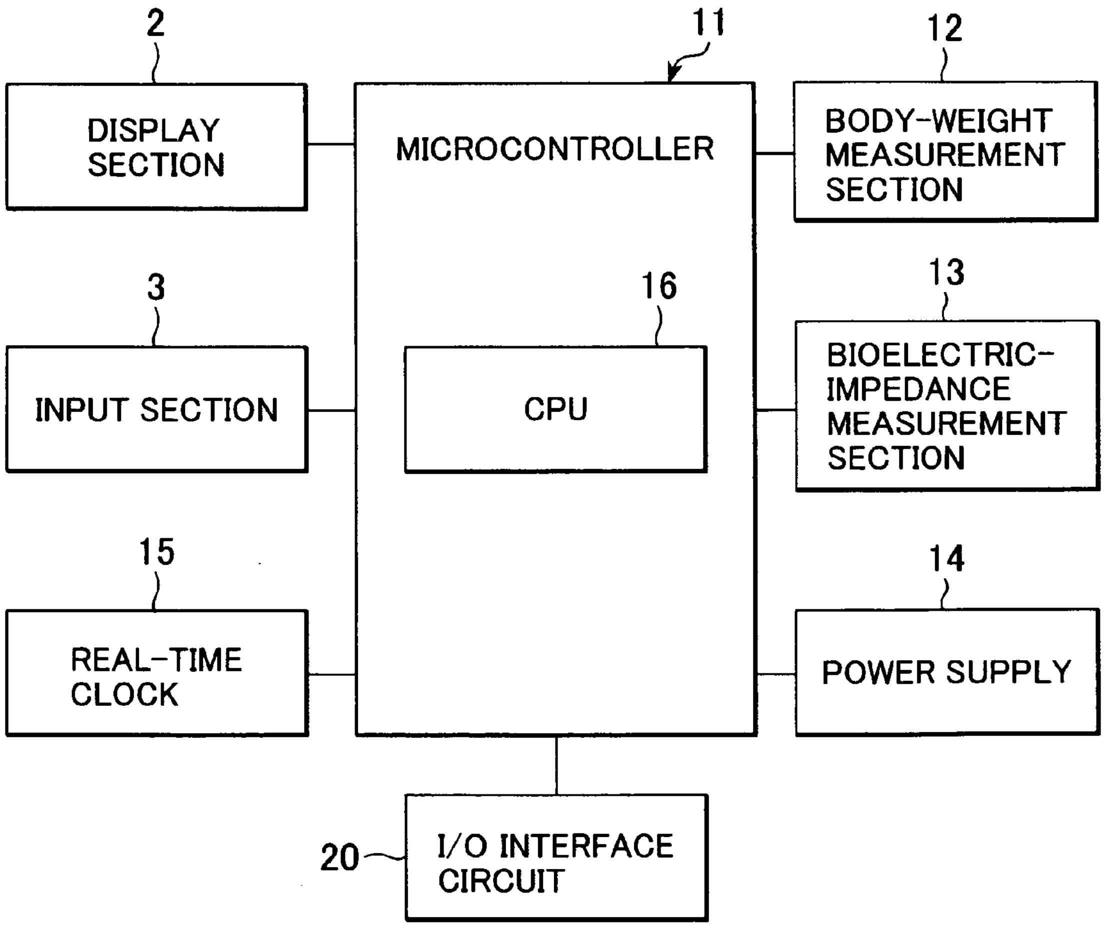
2
DISPLAY SECTION
11
MICROCONTROLLER
12
BODY-WEIGHT MEASUREMENT SECTION
3
INPUT SECTION
16
CPU
13
BIOELECTRIC-IMPEDANCE MEASUREMENT SECTION
15
REAL-TIME CLOCK
14
POWER SUPPLY
20
I/O INTERFACE CIRCUIT

European Patent Office

# EUROPEAN SEARCH REPORT

Application Number
EP 05 02 5787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 275 341 A (TANITA CORPORATION) 15 January 2003 (2003-01-15) | 1,2,9,10 | A61B5/053 |
| Y | * paragraph [0028] - paragraph [0072]; figures * | 5-8, 13-17 | |
| A | EP 1 192 902 A (TANITA CORPORATION) 3 April 2002 (2002-04-03) | 1 | |
| Y | * paragraph [0013] - paragraph [0059]; figures * | 5-8, 13-17 | |

TECHNICAL FIELDS SEARCHED (IPC)

A61B
G06F

The present search report has been drawn up for all claims

2

| Place of search | Date of completion of the search | Examiner |
|---|---|---|
| Munich | 15 March 2006 | Ruff, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 02 5787

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2006

| Patent document cited in search report | | Publication date | Patent family member(s) | | | Publication date |
|---|---|---|---|---|---|---|
| EP 1275341 | A | 15-01-2003 | CN | 1397250 | A | 19-02-2003 |
| | | | JP | 2003088529 | A | 25-03-2003 |
| | | | TW | 564167 | B | 01-12-2003 |
| | | | US | 2003013988 | A1 | 16-01-2003 |
| EP 1192902 | A | 03-04-2002 | JP | 2002102193 | A | 09-04-2002 |
| | | | US | 2003073923 | A1 | 17-04-2003 |

EPO FORM P0459

For more details about this annex : see Official Journal of the European Patent Office, No. 12/82